# EUROPEAN PATENT SPECIFICATION

(11) **EP 1 878 454 B1**
(45) Date of publication and mention of the grant of the patent: **24.04.2019**
(21) Application number: 07111404.5
(22) Date of filing: 29.06.2007
(51) Int. Cl.: A61K 38/33, A61M 5/24, A61M 5/30, A61M 5/31, A61K 38/35, A61M 5/20

(54) **Antihemorrhagic treatment kit**
Anti-hämorrhagischer Behandlungskit
Kit de traitement anti-hémorragique

(30) Priority: 10.07.2006 IT MO20060222
(43) Date of publication of application: 16.01.2008
(73) Proprietor: Health Ricerca E Sviluppo S.R.L., 48124 Ravenna (IT); Noera, Giorgio, 48024 Massalombarda (RA) (IT); Bertolini, Alfio, 42020 Roncolo di Quattro Castella (RE) (IT)
(72) Inventor: Noera, Giorgio, 48024 Massalombarda (Ravenna) (IT); Bertolini, Alfio, 42020 Roncolo di Quattro Castella (RE) (IT)
(74) Representative: Fuochi, Riccardo

(56) References cited:
- WO-A-00/29050
- GB-A- 2 388 033
- US-A- 5 380 710
- US-A1- 2003 036 725
- US-B1- 6 387 078
- US-B1- 6 406 455

## Description

The invention relates to an anti-haemorrhagic dressing kit for administering an anti-haemorrhagic drug, in particular for the first treatment of haemorrhages in the case of traumatic or critical events, for example in road accidents or in the case of wounds in wartime operations.

Critical Mass Events (abbreviation CMA) are defined as the catastrophic events in which the territorial organisation for medical emergencies (abbreviation OME) cannot cope with the front that has been created.

All the resources required in the field are staked in a manner that is directly proportionate to the seriousness and extent of the event.

When treating a patient affected during Critical Mass Events or only during traumatic events, two strategies exist that differ mainly in the dynamics thereof: the first strategy is characterised by the mobility of the patient towards the hospital facility, defined also by the English term "Scoop and Run" - literally scooping up (the body of the patient) and running (to the hospital), abbreviated hereinafter as SaR - and the second strategy uses the mobility of the medical-health professional figure towards the patient, and is also defined by the English term "Stay and Play", literally remain and act on the scene, i.e. transportation is delayed to increase the stability of the patient in the subsequent journey to the hospital facility.

In emergency medicine the sixty minutes period following the occurrence of a traumatic event, or the onset of a serious illness, is called the "Golden Hour". The likelihood of a patient surviving is greater if the medical intervention occurs within the "Golden Hour".

The "Scoop and Run" and "Stay and Play" strategies also differ in terms of cost and type of training of the medical personnel.

Both strategies, in the case of Critical Mass Events, plan the transportation of the patients to hospital facilities: from a quick "Scoop and Run" (SaR), to a first medical treatment of the patient on the scene with the "Stay and Play" strategy.

Both the SaR and "Stay and Play" strategies have advantages and drawbacks, the synthesis of these two opposing strategies has resulted in a new strategy defined by the English term "Play and Run".

The time, which cannot be reduced, - for example the time required for extracting a victim of a road accident from a car - is used for dispensing a first medical treatment.

For example, with the "Play and Run" strategy, the objective of a medical treatment is no longer to restore a normal blood pressure but a minimum blood pressure, by using not only an intravenous infusion but also vasoconstricting drugs or antishock trousers to compress the legs and push the blood into the rest of the body.

The objective is to reduce the risk of death due to the trauma of transportation by trying to comply with the "Golden Hour" rule, i.e. the transportation of the patient to a hospital facility within an hour of the occurrence of the traumatic event.

The "Scoop and Run" strategy (SaR) results to be an unavoidable choice, because of the efficacy which has been proven, for the needs connected to risk factors, to shorter journey times, to the type of pathologies.

For example, in wartime operations under enemy fire ("under fire"), the patient may suffer penetrating wounds for which the immediate transportation to a specialist hospital or a "Trauma Centre", i.e. to a hospital facility dedicated to treating patients affected by serious traumatic pathologies, is necessary.

For this objective it is necessary to achieve extremely short evacuation times, also resorting where possible to the ability to concentrate personnel, means and materials within the "Golden Hour".

The SaR strategy is currently the most used in operation by all forces acting in wartime situations and has largely replaced specialist assistence in situ, also because of the possibility that some harm may not be evident at that moment and may become critical whilst the wounded patient is still very far from the hospital facility.

Nevertheless, even the greatest rapidity and increased skill in the field of medical assistance are not sufficient to ensure the survival of the patient afflicted by serious penetrating injuries.

In fact, the injuries most frequently encountered in the operational area are those caused by bullets, shrapnel, explosions and in general by all those injuries that in the concise jargon of war are known for the sake of brevity by the English term "blast", which are often accompanied by profuse haemorrhages.

The state of haemorrhagic shock following this type of injury, even if the wound is promptly staunched, becomes, according to recent studies, rapidly irreversible and leads to death in up to 40% of patients.

Recent and painful episodes, in out-of-area theatres with high operational intensity have revealed the need to go into this problem thoroughly in order to search for a useful lifesaving instrument for reducing such death incidence following "blast" injuries.

The mortality percentage of critical patients in haemorrhagic shock is directly proportionate to the time (1 hour: 10%; greater than 10 hours: 75%), if they are not properly attended to.

There are two critical points, which are the stabilisation of the patient through reanimation procedures on the scene of the incident and transportation of the patient to equipped and organised units behind the lines.

The degree of efficacy and efficiency of the medical intervention is determined by the existing ratio between the stages and the time through which the number of victims passes and the management thereof offered by the number of available operators, the skills of the latter, the availability and quality of the usable medical treatments, and lastly the steps of logistical organisation.

The prior art with the usual dressing kits gives a result of approximately 40% of patients affected by "blast" wounds who risk dying even if prompt medical assistence was rendered by trained personnel.

In fact, when the haemorrhage has already occurred, unfavourable haemodynamic events connected to the shock are inevitably triggered.

The biological response capacity of the individual is defined as a "survival constant", that counteracts the degree of injury received. The breakdown of this counteraction is the limit threshold in which the victim goes into shock as the sole response prior to death.

The state of shock is characterised by a cascade of biochemical events that intervene up to the non-reversibility degree. The point of no return occurs before the death of the patient and cannot be treated.

Rapid intervention increases the success percentages even if it is not known when and how irreversibility starts. In the medical assistance process this point is an absolute intervention priority that, due to the nature thereof, absorbs the greatest management resources.

The cardiovascular failure that occurs in the shock is due, *inter alias,* to a massive release of endogenous oppioids that act through venodilation, i.e. with stasis of venous blood, which thus does not reach the heart, and also via a depression both of the tone of the sympathetic nervous system and release of the noradrenaline from the nerve endings. This contributes to precipitating an imbalance in the microcircle with stasis of arterial blood even in the capillaries and triggering of a generalised non-bacterial inflammatory reaction with activation of various cells, the release of various chemical substances, amongst which, as a result of enzyme activation, the overproduction of nitric oxide (NO) assumes great importance. The overproduction of nitric oxide (NO) is also responsible for the arterioles' hyporesponsiveness to vasoconstricting agents and for the inhibition of the release of noradrenaline from the nerve endings.

It is therefore necessary to be able to mobilise as soon as possible the remaining residual blood, which is unused in these circumstances because of stasis of the blood. This necessity is a priority in the operating theatre as in the case of wartime events, critical mass events, etc.

U S 5,380,710 discloses a pharmaceutical composition containing ACTH (1-24) for the therapy of shock conditions, which may be administered with an injection kit.

US 2003/0036725 discloses a reconstitution and injection system for delivering a drug under pressure, suitable to inject powdered or lyophilised drugs that require reconstitution, rehydration or diluition. Such reconstruction and injection system comprises an housing having a first recessed port or opening for receiving a syringe with a diluent, and a second recessed port or opening for receiving a drug cartridge or vial containing a lyophilised drug or compound concentrate. The communication between the syringe, containing the diluent, and the vial, containing the lyophilised drug, is regulated by a control button.

US 6,406,455 discloses an injection device comprising a reservoir for a lyophilised drug, a fluid reservoir containing an aqueous diluent bounded by two pistons, and a fixed actuator. Liquid pathways are provided between the fluid reservoir and the lyophilised drug reservoir.

US 6,387,078 discloses an automatic mixing and injecting apparatus comprises a syringe assembly within a housing. The syringe assembly has a chamber for holding a liquid, which can be a liquid medicine or a solvent; a second chamber holds a dry medicine and is releasably sealed with respect to the first chamber. A spring-operated plunger forces liquid from the first chamber and causes the releasable seal to disengage when the needle has entered the recipient. At this time, the liquid flows through the second chamber and dissolves any dry medicine in that chamber. A releasable coupling disengages the plunger from the driver spring and allows the plunger, syringe, and needle to retract under the urging of a return spring.

An object of the invention is to improve known anti-haemorrhagic dressing kits.

Another object of the invention is to present an anti-haemorrhagic dressing kit that is easy to use, also by non-specialised personnel.

Still another object is to present an anti-haemorrhagic dressing kit that is cheap to produce.

According to an aspect of the present invention, an anti-haemorrhagic dressing kit is presented as specified in claims 1 and 2.

Owing to the form of application with an autoinjector, the dressing kit according to the invention can also be used by non-medical or nursing personnel.

Further, the use of an active principle deriving from the adrenocorticotropic hormone (ACTH) enables the patient in shock to be stabilised for a period of at least 3 hours and thus the reversibility of the shock to be moved by at least 2 hours.

The invention can be better understood and implemented with reference to the attached drawings, which illustrate an embodiment thereof by way of non-illustrative example, in which:
Figure 1 is a side view of a main component of the anti-haemorrhagic dressing kit according to the invention;
Figure 2 is a section of the main component in figure 1;
Figure 3 is a section of another embodiment of a component of the anti-haemorrhagic dressing kit according to the invention; and Figure 4 is a section of the component of Figure 3 after an injection has been administered.

The dressing kit according to the invention is contained in a package (not shown), which comprises an autoinjector filled with an antishock drug and a leaflet (not shown) with written instructions on administering the antishock drug.

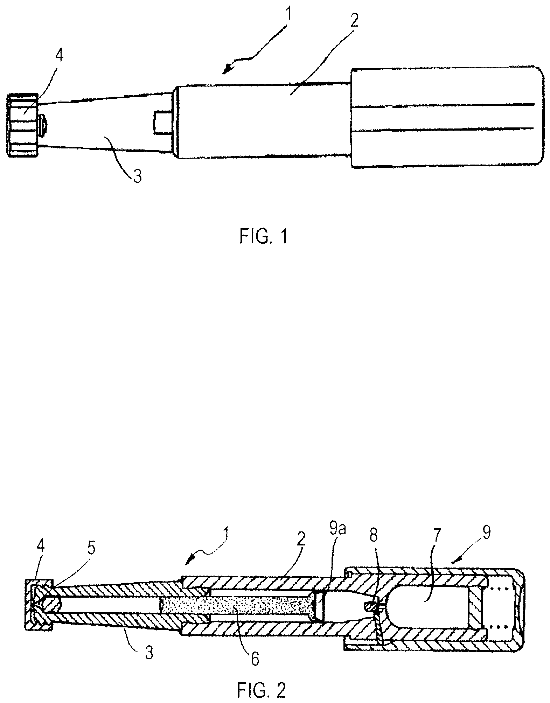

According to what has been shown in Figures 1 and 2, a first embodiment of the autoinjector 1 is without a needle and comprises a main body 2, a container 3 containing an antishock drug in liquid form, a closing cap 4 for a nozzle 5 connected to the container 3.

The nozzle 5 has to be positioned in direct contact with the skin of the patient and owing to the high speed at which the drug is caused to pass through the nozzle 5, the drug inside the container 3 is able to pass through the skin of the patient.

The drug is therefore injected into the body of the patient also without a needle.

In the container 3 a piston 6 is provided that is driven by actuating means 9 comprising pneumatic means that is provided with a chamber 7 with an inert pressurised gas. By interrupting a dividing baffle 8, the first chamber 7 with the pressurised gas is made to communicate with an actuator 9a connected to the piston 6. The piston 6 is then driven at high speed by the gas of the chamber 7 and consequently also the drug is caused to pass through the nozzle 5 at high speed.

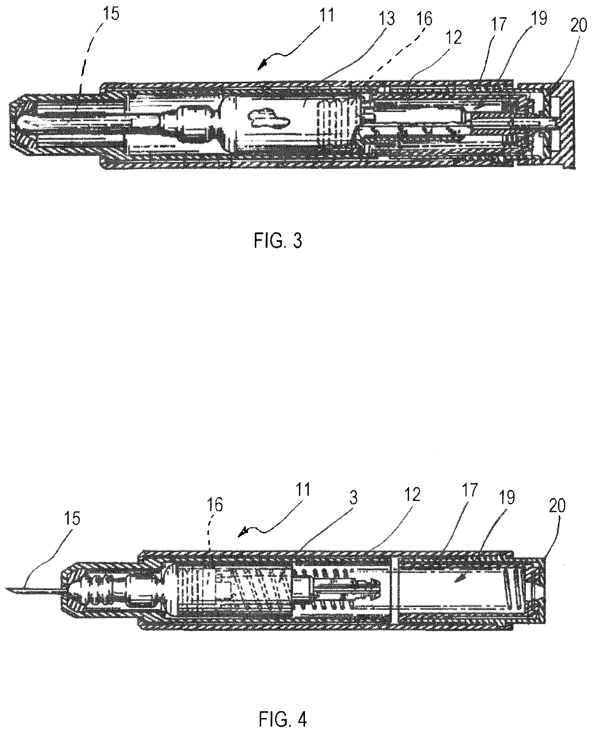

In Figures 3 and 4 there is shown an autoinjector 11 in the embodiment provided with a needle 15. The autoinjector 11 comprises an external casing 12 that is able to receive the needle 15 in a retracted position, a container 13 connected to the needle 15 and containing an antishock drug, the autoinjector 11 further comprises actuating means 19 for actuating the injection.

The actuating means 19 comprises elastic means 17 that first causes the needle 15 emerge and then injects the antishock drug by driving a piston 16 acting inside the container 13.

In Figure 3 the autoinjector 11 is ready to administer an injection of the antishock drug inside the container 13. In Figure 4 the autoinjector 11 is shown in an administered injection condition, with the needle 15 extracted and the container 13 emptied.

In a further embodiment of the invention (not shown) that can be used both with autoinjectors with needles and with autoinjectors without needles, the pharmacological part is lyophilised and it is thus possible to have a long storage life (about 60 months).

In this embodiment, the lyophilised pharmacological part is kept in the first container separated by the second container in which there is the liquid part of the drug. At the moment of use of the autoinjector, the actuating means 9, 19 also connects the first and second container, for example by interrupting a dividing wall, and mixing the lyophilised pharmacological part with the liquid part.

In this way, the antishock drug is formed only at the moment of use and can be stored for a long time even in unfavourable climatic conditions, for example at a high ambient temperature.

In use, both types of autoinjector 1, 11 have to be placed in direct contact with the skin of the patient and are maintained in contact through moderate pressure until the injection has been administered. In order to activate the autoinjectors 1, 11, it may be necessary to remove a safety element and thus drive a control 10, 20.

According to the invention, the antishock drug used in the autoinjectors 1, 11 contains as an active principle a polypeptide selected from a group comprising the amino acid sequence 1-24 of the adrenocorticotropic hormone (ACTH 1-24) and all the fragments and analogues thereof, and analogues of fragments, with agonist activity on the MC4 melanocortin receptors, i.e. a polypeptide that binds with the MC4 melanocortin receptors with a consequent biochemical and/or physiological response, and all the synthetic agonists, also with a non-peptide structure, of the MC4 receptors for the melanocortins. In particular, the active principle may be tetracosactide esacetate.

It must be stated beforehand that the antishock effects of the melanocortin peptides are not mediated by the adrenal glands (cortisonics) as these effects are obtained experimentally both with melanocortin preparations devoid of corticotropic activity and in animals the adrenal glands of which have been removed.

The antishock effect of the melanocortins, peptides that bind with specific receptors in the central nervous system, the MC4 receptors, is expressed by restoring that vasomotor reflex which is made inactive by the sequestration of peripheral blood due to the massive release of noradrenaline and the activation of the inflammatory cascade until enzyme activation and production of nitric oxide, following the secretion of oppioids from the second phase to the irreversible shock phase.

This restoration occurs through the action counteracting the oppioids and the nitric oxide.

The melanocortins release the blood that was previously removed from circulation through stasis of the blood, also making this quantity of blood available.

The blood that is thus brought back into circulation is particularly precious because it is used as if it were a blood reserve in the organism of the patient that is devoid of blood due to the haemorrhage.

It is thus possible to move the reversibility curve of the shock by using one of the aforesaid antishock drugs, for example the tetracosactide esacetate, because it is able to counteract with a single, effective and low-cost treatment the time function in the non-hospital care process by intervening significantly at all the mentioned critical points.

The dose of drug for a single shock treatment is 10 mg, and enables the patient in shock to be stabilised for a period of at least 3 hours and thus it enables the reversibility of the shock to be moved by at least 2 hours.

The patient may lose up to 50-60 % of circulatory blood mass in this period, noticeably increasing the survival index up to 90%.

The drug, as mentioned above, is injected intramuscularly and intravenously (intralingually and/or intraosseously) through the autoinjector 1, 11, and the action thereof occurs after between 5 and 15 minutes.

The drug according to the invention is not toxic and can also be administered to healthy people without side effects; owing to the form of application with autoinjector the drug can also be used by non-medical or nursing personnel.

The drug according to the invention can replace treatment by infusion of 1-3 litres of liquids or blood that are normally used in these cases.

The advantages over the medical treatments currently in use in the case of haemorrhage are:
- low production costs and high clinical efficacy in all types of shock;
- lower cost compared with blood, storage and supply;
- lower cost than a volume expander;
- low transportation cost and logistic availability;
- no need for medical skills;
- possibility of using an autoinjector;
- in the embodiment with a lyophilised pharmacological part it is possible to preserve the dressing kit for a long period of time, for example about 60 months.

## Claims

1. Anti-haemorrhagic dressing kit for administering an anti-haemorrhagic drug, in particular for treating haemorrhages of traumatic origin in emergency situations, comprising a drug with an active principle selected from the group comprising the amino acid sequence 1-24 of the adrenocorticotropic hormone (ACTH 1-24) and all the fragments and analogues thereof, and analogues of fragments, with agonist activity on the MC4 melanocortin receptors, and all the synthetic agonists, also having non-peptide structure, of the MC4 receptors for the melanocortins, **characterised in that** said anti-haemorrhagic dressing kit comprises an autoinjector (1) for automatically injecting said drug into a patient, said autoinjector (1) being without a needle and comprising a main body (2), a container (3) containing the antishock drug, a closing cap (4) for a nozzle (5) connected to the container (3); in the container (3) is provided a piston (6) that is driven by actuating means (9) comprising pneumatic means that is provided with a chamber (7) with an inert pressurised gas and a dividing baffle (8); said autoinjector (1) also comprising a first container for containing the lyophilised pharmacological part of the antishock drug and a second container for containing the liquid part of the drug, said first and second container being divided by a dividing wall; only at the moment of use the actuating means (9, 19) connects the first and second container by interrupting a dividing wall, mixing the lyophilised pharmacological part with the liquid part.

2. Anti-haemorrhagic dressing kit for administering an anti-haemorrhagic drug, in particular for treating haemorrhages of traumatic origin in emergency situations, comprising a drug with an active principle selected from the group comprising the amino acid sequence 1-24 of the adrenocorticotropic hormone (ACTH 1-24) and all the fragments and analogues thereof, and analogues of fragments, with agonist activity on the MC4 melanocortin receptors, and all the synthetic agonists, also having non-peptide structure, of the MC4 receptors for the melanocortins, **characterised in that** said anti-haemorrhagic dressing kit comprises an autoinjector (11) for automatically injecting said drug into a patient, said autoinjector (11) being provided with a needle (15) and comprising an external casing (12) that is able to receive the needle (15) in a retracted position, a container (13) connected to the needle (15) and containing the antishock drug; the autoinjector (11) further comprising actuating means (19) for actuating the injection; the actuating means (19) comprising a single elastic means (17) that first causes the needle (15) to emerge and then injects the antishock drug by driving a piston (16) acting inside the container (13); said autoinjector (1) also comprising a first container for containing the lyophilised pharmacological part of the antishock drug and a second container for containing the liquid part of the drug, said first and second container being divided by a dividing wall; only at the moment of use the actuating means (19) connects the first and second container by interrupting a dividing wall, mixing the lyophilised pharmacological part with the liquid part.

3. Kit according to claim 2, wherein said needle (15) is normally in a retracted position and is in an extracted position in the condition of administering the injection.

4. Kit according to claim 3, wherein said actuating means (19) extracts said needle (15).

5. Kit according to any preceding claim, wherein said anti-haemorrhagic drug comprises tetracosactide esacetate.

6. Package comprising a kit according to any preceding claim and a sheet with written instructions for administering the antishock drug.

## Patentansprüche

1. Antihämorrhagisches Verbandset zur Verabreichung eines antihämorrhagischen Arzneimittels, insbesondere zur Behandlung von Blutungen traumatischen Ursprungs in Notsituationen, umfassend ein Arzneimittel mit einem Wirkstoff ausgewählt aus der Gruppe umfassend die Aminosäuresequenz 1-24 des adrenokortikotropen Hormons (ACTH 1-24) und alle Fragmente und Analoga davon, sowie Analoga von Fragmenten, mit agonistischer Aktivität auf die MC4-Melanocortin-Rezeptoren, und alle synthetischen Agonisten, auch mit nicht-peptidischer Struktur, der MC4-Rezeptoren für die Melanocortine, **dadurch gekennzeichnet, dass** das besagte antihämorrhagische Verbandset einen Autoinjektor (1) für das automatische Injizieren des besagten Arzneimittels in einen Patienten umfasst, wobei der besagte Autoinjektor (1) ohne Nadel ist und einen Hauptkörper (2), einen Behälter (3), der das Antischock-Arzneimittel enthält, eine Verschlusskappe (4) für eine Düse (5), die mit dem Behälter (3) verbunden ist, umfasst; in dem Behälter (3) ist ein Kolben (6) vorgesehen, der durch ein Betätigungsmittel (9) angetrieben wird, das ein pneumatisches Mittel umfasst, das mit einer Kammer (7) mit einem inerten Druckgas und einer Trennwand (8) versehen ist; der besagte Autoinjektor (1) ebenfalls umfassend einen ersten Behälter zum Aufnehmen des lyophilisierten pharmakologischen Teils des Antischock-Arzneimittels und einen zweiten Behälter zum Aufnehmen des flüssigen Teils des Arzneimittels, wobei der besagte erste und zweite Behälter durch eine Trennwand getrennt sind; erst zum Zeitpunkt der Verwendung verbindet das Betätigungsmittel (9, 19) den ersten und zweiten Behälter durch Unterbrechen einer Trennwand, wodurch der lyophilisierte pharmakologische Teil mit dem flüssigen Teil gemischt wird.

2. Antihämorrhagisches Verbandset zur Verabreichung eines antihämorrhagischen Arzneimittels, insbesondere zur Behandlung von Blutungen traumatischen Ursprungs in Notsituationen, umfassend ein Arzneimittel mit einem Wirkstoff ausgewählt aus der Gruppe umfassend die Aminosäuresequenz 1-24 des adrenokortikotropen Hormons (ACTH 1-24) und alle Fragmente und Analoga davon, sowie Analoga von Fragmenten, mit agonistischer Aktivität auf die MC4-Melanocortin-Rezeptoren, und alle synthetischen Agonisten, auch mit nicht-peptidischer Struktur, der MC4-Rezeptoren für die Melanocortine, **dadurch gekennzeichnet, dass** das besagte antihämorrhagische Verbandset einen Autoinjektor (11) für das automatische Injizieren des besagten Arzneimittels in einen Patienten umfasst, wobei der besagte Autoinjektor (11) mit einer Nadel (15) versehen ist und ein Außengehäuse (12), das die Nadel (15) in einer zurückgezogenen Position aufnehmen kann, einen Behälter (13), der mit der Nadel (15) verbunden ist und das Antischock-Arzneimittel enthält, umfasst; der Autoinjektor (11) ferner umfassend ein Betätigungsmittel (19) zum Betätigen der Injektion; das Betätigungsmittel (19) umfassend ein einzelnes elastisches Mittel (17), das zunächst bewirkt, dass die Nadel (15) austritt und dann das Antischock-Arzneimittel durch Antreiben eines Kolbens (16), der innerhalb des Behälters (13) wirkt, injiziert; der besagte Autoinjektor (1) ebenfalls umfassend einen ersten Behälter zum Aufnehmen des lyophilisierten pharmakologischen Teils des Antischock-Arzneimittels und einen zweiten Behälter zum Aufnehmen des flüssigen Teils des Arzneimittels, wobei der besagte erste und zweite Behälter durch eine Trennwand getrennt sind; erst zum Zeitpunkt der Verwendung verbindet das Betätigungsmittel (19) den ersten und zweiten Behälter durch Unterbrechen einer Trennwand, wodurch der lyophilisierte pharmakologische Teil mit dem flüssigen Teil gemischt wird.

3. Set nach Anspruch 2, worin sich die besagte Nadel (15) normalerweise in einer zurückgezogenen Position und in einer ausgezogenen Position im Zustand der Verabreichung der Injektion befindet.

4. Set nach Anspruch 3, worin das besagte Betätigungsmittel (19) die besagte Nadel (15) auszieht.

5. Set nach irgendeinem vorhergehenden Anspruch, worin das antihämorrhagische Arzneimittel Tetracosactid hexaacetat umfasst.

6. Verpackung, umfassend ein Set nach irgendeinem vorhergehenden Anspruch und ein Blatt mit schriftlichen Anweisungen für die Verabreichung des Antischock-Arzneimittels.

## Revendications

1. Kit de pansements anti-hémorragiques pour administrer un médicament anti-hémorragique, en particulier pour traiter les hémorragies d'origine traumatique dans des situations d'urgence, comprenant un médicament avec un principe actif sélectionné parmi le groupe comprenant la séquence d'acides aminés 1-24 de l'hormone corticotrope (ACTH 1-24) et tous les fragments et analogues de celle-ci, et analogues de fragments, avec une activité agoniste sur les récepteurs de la mélanocortine MC4, et tous les agonistes synthétiques, ayant également une structure non peptidique, des récepteurs MC4 pour les mélanocortines, **caractérisé en ce que** ledit kit de pansements anti-hémorragiques comprend un auto-injecteur (1) pour injecter automatiquement ledit médicament dans un patient, ledit auto-injecteur (1) étant dépourvu d'aiguille et comprenant un corps principal (2), un récipient (3) contenant le médicament antichoc, un bouchon (4) pour un embout (5) raccordé au récipient (3) ; le récipient (3) contient un piston (6) entraîné par des moyens d'actionnement (9) comprenant des moyens pneumatiques dotés d'une chambre (7) avec un gaz inerte sous pression et un déflecteur de séparation (8) ; ledit auto-injecteur (1) comprenant également un premier récipient pour contenir la partie pharmacologique lyophilisée du médicament antichoc et un deuxième récipient pour contenir la partie liquide du médicament, lesdits premier et deuxième récipients étant divisés par une cloison de séparation ; c'est seulement au moment de l'utilisation que les moyens d'actionnement (9, 19) raccordent le premier et le deuxième récipients en interrompant une cloison de séparation, en mélangeant la partie pharmacologique lyophilisée avec la partie liquide.

2. Kit de pansements anti-hémorragiques pour administrer un médicament anti-hémorragique, en particulier pour traiter les hémorragies d'origine traumatique dans des situations d'urgence, comprenant un médicament avec un principe actif sélectionné parmi le groupe comprenant la séquence d'acides aminés 1-24 de l'hormone corticotrope (ACTH 1-24) et tous les fragments et analogues de celle-ci, et analogues de fragments, avec une activité agoniste sur les récepteurs de la mélanocortine MC4, et tous les agonistes synthétiques, ayant également une structure non peptidique, des récepteurs MC4 pour les mélanocortines, **caractérisé en ce que** ledit kit de pansements anti-hémorragiques comprend un auto-injecteur (11) pour injecter automatiquement ledit médicament dans un patient, ledit auto-injecteur (11) étant doté d'une aiguille (15) et comprenant une enveloppe extérieure (12) capable de recevoir l'aiguille (15) dans une position rétractée, un récipient (13) raccordé à l'aiguille (15) et contenant le médicament antichoc ; l'auto-injecteur (11) comprenant également des moyens d'actionnement (19) pour actionner l'injection ; les moyens d'actionnement (19) comprenant un moyen élastique unique (17) qui fait d'abord sortir l'aiguille (15) et injecte ensuite le médicament antichoc en entraînant un piston (16) agissant dans le récipient (13) ; ledit auto-injecteur (1) comprenant également un premier récipient pour contenir la partie pharmacologique lyophilisée du médicament antichoc et un deuxième récipient pour contenir la partie liquide du médicament, lesdits premier et deuxième récipients étant divisés par une cloison de séparation ; c'est seulement au moment de l'utilisation que les moyens d'actionnement (19) raccordent le premier et le deuxième récipients en interrompant une cloison de séparation, en mélangeant la partie pharmacologique lyophilisée avec la partie liquide.

3. Kit selon la revendication 2, dans lequel ladite aiguille (15) est normalement dans une position rétractée et est dans une position saillante dans l'état d'administration de l'injection.

4. Kit selon la revendication 3, dans lequel lesdits moyens d'actionnement (19) extraient ladite aiguille (15) .

5. Kit selon l'une des revendications précédentes, dans lequel ledit médicament anti-hémorragique comprend de l'hexacétate de tétracosactide.

6. Emballage comprenant un kit selon l'une des revendications précédentes et une feuille avec des instructions écrites pour administrer le médicament antichoc.
